# FASCICULE DE BREVET EUROPEEN

(11) **EP 1 616 554 B1**
(45) Date de publication et mention de la délivrance du brevet: **29.04.2009**
(21) Numéro de dépôt: 05291244.1
(22) Date de dépôt: 09.06.2005
(51) Int. Cl.: A61K 8/06, A61K 8/37, A61K 8/92, A61K 8/39, A61K 8/34, A61Q 1/00, A61Q 5/00, A61Q 17/00, A61Q 19/00

(54) **Emulsion H/E fine et son utilisation dans le domaine cosmétique**
Feine Ö/W-Emulsion und ihre Verwendung im Kosmetikbereich
Fine O/W-emulsion and its use in cosmetic fields

(30) Priorité: 16.07.2004 FR 0451547
(43) Date de publication de la demande: 18.01.2006
(73) Titulaire: L'ORÉAL, 75008 Paris (FR)
(72) Inventeur: Sebillotte-Arnaud, Laurence, 94240 L'Hay les Roses (FR); Aubrun, Odile, 92160 Antony (FR)
(74) Mandataire: Rasson, Catherine

(56) Documents cités:
- DE-A1- 19 532 543
- US-A- 4 606 913
- US-A- 6 086 787
- US-A1- 2003 181 602
- US-B1- 6 221 370
- US-B1- 6 623 746

## Description

La présente invention se rapporte à une composition pour application topique, notamment cosmétique et/ou dermatologique, sous forme d'émulsion fine huile-dans-eau, riche en huile, susceptible d'être obtenue par inversion de phase, et à ses utilisations notamment dans le domaine cosmétique ou dermatologique.

Il est connu d'utiliser dans le domaine cosmétique ou dermatologique, des émulsions huile-dans-eau (H/E). Ces émulsions qui sont constituées d'une phase huileuse (ou phase lipophile) dispersée dans une phase aqueuse ont une phase aqueuse externe et sont donc des produits plus agréables à utiliser, en raison de la sensation de fraîcheur qu'elles procurent. Toutefois, elles présentent l'inconvénient de manquer relativement de stabilité quand la quantité d'huile présente est trop importante. Or, pour certaines applications, il est avantageux de disposer d'un grande quantité d'huiles car les huiles apportent du confort à la peau, la nourrissent et peuvent aussi la démaquiller quand ces huiles ont des propriétés démaquillantes.

Par ailleurs, il est avantageux de disposer d'émulsions fines, c'est-à-dire d'émulsions où la phase huileuse se trouve sous forme de très petites gouttelettes, c'est-à-dire de gouttelettes de taille inférieure à 4 µm, car ces émulsions fines ont un toucher cosmétique agréable et sont en général plus stables que les émulsions grossières.

Ces émulsions peuvent être préparées notamment par la technique d'inversion de phase en température (émulsions PIT), dans lesquelles la taille moyenne des globules constituant la phase huileuse est comprise dans des limites déterminées, à savoir entre 0,1 à 4 µm (100 à 4000 nm). Le principe d'émulsification par inversion de phase en température (en Anglais : Phase Inversion Temperature ou PIT) est, dans son principe, bien connu de l'homme de l'art ; il a été décrit en 1968 par K. Shinoda (J. Chem. Soc. Jpn., 1968, 89, 435). Il a été montré que cette technique d'émulsification permet d'obtenir des émulsions fines stables (K. Shinoda et H. Saito, J. Colloïd Interface Sci., 1969,30, 258). Cette technologie a été appliquée en cosmétique dès 1972 par Mitsui et al. («Application of the phase-inversion-temperature method to the emulsification of cosmetics» ; T. Mitsui, Y. Machida and F. Harusawa, American. Cosmet. Perfum., 1972, 87, 33).

Le principe de cette technique est le suivant : on prépare une émulsion E/H (introduction de la phase aqueuse dans la phase huileuse) à une température qui doit être supérieure à la température d'inversion de phase du système, c'est à dire la température à laquelle l'équilibre entre les propriétés hydrophile et lipophile du ou des émulsionnants mis en oeuvre est atteint ; à température élevée, c'est-à-dire supérieure à la température d'inversion de phase (>PIT), l'émulsion est de type eau-dans-huile, et, au cours de son refroidissement, cette émulsion s'inverse à la température d'inversion de phase, pour devenir une émulsion de type huile-dans-eau, et ceci en étant passée auparavant par un état de microémulsion. Ce procédé permet d'obtenir facilement des émulsions de diamètre généralement inférieur à 4 µm. Les tensioactifs émulsionnants du type huile-dans-eau habituellement utilisés ont un HLB (HLB = Hydrophilic Lipophilic Balance) allant de 8 à 18. Ces émulsionnants, grâce à leur structure amphiphile, se placent à l'interface phase huileuse / phase aqueuse, et stabilisent ainsi les gouttelettes d'huiles dispersées.

Toutefois, il est difficile de faire des émulsions H/E fines contenant une quantité importante de phase huileuse, car de telles émulsions ont tendance à se déstabiliser, cette déstabilisation se traduisant par une coalescence et une séparation des phases aqueuse et huileuse avec un relargage de l'huile. Pour améliorer la stabilité de ces émulsions, on peut augmenter la concentration en émulsionnants ; toutefois, une forte concentration en émulsionnants peut conduire à un toucher rêche, collant ou poisseux, ainsi qu'à des problèmes d'innocuité vis-à-vis de la peau, des yeux et du cuir chevelu.

Il est décrit dans le document WO-A-01/89678 des émulsions riches en huile, comportant 70 % d'huile, cette huile étant du dicaprylyl éther. Toutefois, la stabilité de ces émulsions n'est pas suffisante, comme le montrent les exemples comparatifs présentés plus loin , notamment l'exemple comparatif 1.

Il subsiste donc le besoin de disposer d'émulsions H/E fines contenant une quantité importante d'huiles tout en étant stables.

La demanderesse a trouvé de manière surprenante que le choix de certaines huiles à des taux particuliers permettait d'obtenir des émulsions fines stables, ayant en outre de bonnes propriétés cosmétiques.

La présente invention a donc pour objet une composition pour application topique sous forme d'émulsion huile-dans-eau, caractérisée par le fait qu'elle comporte :
- Une phase lipophile (A) présente en une quantité d'au moins 50 % en poids par rapport au poids total de la composition, la phase lipophile comprenant au moins 25 % en poids d'une ou plusieurs huiles hydrocarbonées synthétiques ou minérales, ayant un poids moléculaire supérieur ou égal à 360 g/mole, par rapport au poids total de la phase lipophile,
- une phase aqueuse (C) présente en une quantité inférieure ou égale à 45 % en poids par rapport au poids total de la composition,
- un système émulsionnant (B) présent en une quantité de 2 à 20 % en poids par rapport au poids total de la composition et comprenant au moins un émulsionnant ayant un HLB allant de 8 à 18, choisi parmi les alcools gras éthoxylés, les acides gras éthoxylés, les glycérides partiels d'acides gras ethoxylés, les triglycérides d'acides gras polyglycérolés et leurs dérivés éthoxylés, et leurs mélanges,
- le rapport système émulsionnant (B) / phase lipophile (A) allant de 0,04 à 0,2.

Du fait qu'elle est destinée à une application topique, la composition de l'invention contient un milieu physiologiquement acceptable. Par "milieu physiologiquement acceptable", on entend un milieu convenant à une application topique sur la peau ou les phanères, c'est-à-dire compatible avec la peau, les muqueuses, les lèvres, les cils, les yeux, les cheveux et les ongles. Cette composition peut constituer notamment une composition cosmétique ou dermatologique.

On entend par « phase lipophile » dans la présente demande, la phase contenant les composés lipophiles que sont notamment les huiles (constituants lipophiles liquides à température ambiante), les gommes, les pâtes et les cires. Ce sont par exemple des triglycérides, des hydrocarbures, des esters, des éthers, des silicones, comme décrit plus loin, et tous les additifs lipophiles éventuellement présents. Les émulsionnants et co-émulsionnants du système émulsionnant ne font pas partie de la phase lipophile telle que définie ci-dessus.

Il est important d'avoir une quantité suffisante de phase lipophile et notamment d'huiles pour obtenir une texture crémeuse, et le problème à la base de l'invention a été la difficulté d'obtenir une composition de texture crémeuse avec des petites tailles de globules, qui contienne suffisamment d'huiles tout en étant néanmoins bien stable.

Selon un mode préféré de l'invention, la composition comprend en outre de préférence au moins un polyol comme décrit de manière plus détaillée ci-après.

Les émulsions H/E selon l'invention sont généralement obtenues par la technologie d'inversion de phase en température et se caractérisent par :
- leur viscosité : il s'agit principalement de crèmes,
- leur aspect qui peut aller de l'opaque au translucide,
- leur pH qui va de 3 à 8,
- la petite taille des gouttelettes de la phase huileuse,
- leur stabilité : la variation de viscosité après deux mois 45°C est inférieure ou égale à 33 % par rapport au temps 24h à température ambiante (20-25°C).

Ainsi, les émulsions obtenues selon l'invention, malgré la quantité importante d'huiles qu'elles contiennent, sont stables. On entend par « composition stable » une composition qui reste macroscopiquement homogène après 2 mois à 45°C et dont la variation de viscosité (en plus ou en moins) par rapport à la viscosité initiale est inférieure ou égale à 33 % (mesure au Rhéomat 180). Une telle stabilité signifie qu'il ne se produit ni déphasage macroscopique ni changement de texture telle que l'apparition de grains, après ce laps de temps.

Les compositions selon l'invention peuvent se présenter sous forme de crèmes plus ou moins épaisses, opaques à translucides, et elles peuvent s'écouler ou non sous leur propre poids selon leur viscosité. Pour une crème, la viscosité mesurée à 25°C avec l'appareil de mesure Rheomat 180 à 200 rpm (tours par minute) doit être égale ou supérieure à 1 Pa.s. Le Rheomat 180 est équipé d'un mobile différent selon les viscosités, par exemple d'un mobile 3 pour la gamme des viscosités de 0,2 à 4 Pa.s, et d'un mobile 4 pour la gamme des viscosités supérieures à 2 Pa.s. Mesurée dans les conditions indiquées ci-dessus, la viscosité des compositions de l'invention peut aller par exemple de 1 à 30 Pa.s et de préférence de 1 à 20 Pa.s. Cette viscosité est mesurée généralement 10 minutes après la mise en rotation du mobile.

La taille moyenne des gouttelettes de phase huileuse sont mesurées par diffraction de la lumière à l'aide d'un granulomètre Mastersizer 2000 (commercialisés par Malvern Instruments). Ces mesures sont effectuées sur l'émulsion diluée dans une solution de SDS (Sodium Dodecylsulfate) à 1 % dans l'eau. Un logiciel permet d'accéder au diamètre moyen en volume D[4,3] (µm) (Voir Operators Guide, Malvern Instruments, Decembre 1998, p.61 à 67).

La taille moyenne D[4,3] (µm) des gouttelettes de phase huileuse de la composition de l'invention varie de 0,09 µm à 4 µm, plus particulièrement de 0,1 µm à 2 µm et de préférence de 0,1 µm à 1 µm.

### Système émulsionnant

Le système émulsionnant (B) utilisé dans la composition selon l'invention comprend un ou plusieurs émulsionnants dont la solubilité dans l'huile augmente avec l'augmentation de la température, émulsionnants permettant d'obtenir des émulsions par inversion de phase en température. Le HLB (hydrophilic lipophilic balance) de ces émulsionnants va de 8 à 18 et de préférence de 10 à 16, et ces émulsionnants sont choisis parmi les alcools gras éthoxylés, les acides gras éthoxylés, les glycérides partiels d'acides gras éthoxylés, les triglycérides d'acides gras polyglycérolés et leurs dérivés éthoxylés, et leurs mélanges.

Les émulsionnants sont de préférence choisis parmi les alcools gras éthoxylés ou les acides gras éthoxylés ayant les formules (I) et (II) suivantes :

R-O-(CH₂-CH₂-O)ₘH (I)

R-COO-(CH₂-CH₂-O)ₘH (II)

où R est une chaîne hydrocarbonée linéaire ou ramifiée, saturée ou insaturée, ayant de 10 à 24 atomes de carbone, et m est un nombre entier allant de 8 à 50.

Comme alcools gras éthoxylés, on peut citer par exemple les produits d'addition d'oxyde d'éthylène avec l'alcool laurylique, notamment ceux comportant de 9 à 50 groupes oxyéthylénés (Laureth-9 à Laureth-50 en noms CTFA) ; les produits d'addition d'oxyde d'éthylène avec l'alcool béhénylique, notamment ceux comportant de 9 à 50 groupes oxyéthylénés (Beheneth-9 à Beheneth-50 en noms CTFA) ; les produits d'addition d'oxyde d'éthylène avec l'alcool cétéarylique (mélange d'alcool cétylique et d'alcool stéarylique), notamment ceux comportant de 9 à 30 groupes oxyéthylénés (Ceteareth-9 à Ceteareth-30 en noms CTFA) ; les produits d'addition d'oxyde d'éthylène avec l'alcool cétylique, notamment ceux comportant de 9 à 30 groupes oxyéthylénés (Ceteth-9 à Ceteth-30 en noms CTFA) ; les produits d'addition d'oxyde d'éthylène avec l'alcool stéarylique, notamment ceux comportant de 9 à 30 groupes oxyéthylénés (Steareth-9 à Steareth-30 en noms CTFA) ; les produits d'addition d'oxyde d'éthylène avec l'alcool isostéarylique, notamment ceux comportant de 9 à 50 groupes oxyéthylénés (Isosteareth-9 à Isosteareth-50 en noms CTFA); et leurs mélanges.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA, PEG-9 palmitate à PEG-50 palmitate) ; les stéarates de PEG-9 à PEG-50 (en noms CTFA : PEG-9 stearate à PEG-50 stéarate) ; les palmito-stéarates de PEG-9 à PEG-50 ; les béhénates de PEG-9 à PEG-50 (en noms CTFA : PEG-9 behenate à PEG-50 behenate), et leurs mélanges.

On peut utiliser aussi des mélanges de ces dérivés oxyéthylénés d'alcools gras et d'acides gras.

De préférence, le système émulsionnant de la composition de l'invention contient comme émulsionnant au moins un alcool gras éthoxylé, et plus particulièrement le beheneth-10.

Le système émulsionnant peut contenir en outre un ou plusieurs co-émulsionnants. Comme co-émulsionnants, on peut citer par exemple les alcools gras ayant 8 à 30 atomes de carbone, comme par exemple l'alcool cétylique, l'alcool stéarylique, l'alcool béhénique ; les acides gras ayant 8 à 30 atomes de carbone, comme par exemple l'acide palmitique, l'acide stéarique, l'acide béhénique ; les esters gras de glycérol, comme par exemple le glycéryl stéarate ; les dérivés oxyéthylénés de ces alcools gras, acides gras et esters gras de glycérol, comportant 2 à 8 groupes oxyde d'éthylène, et leurs mélanges.

Le système émulsionnant est présent en une quantité allant de 2 à 20 %, de préférence de 3 à 16 % et mieux de 3 à 11 % en poids par rapport au poids total de la composition.

Le rapport système émulsionnant (B) / phase lipophile (A) va de 0,04 à 0,2, de préférence de 0,06 à 0,18. Comme indiqué plus haut, on entend par « phase lipophile » l'ensemble des constituants qui ne sont pas hydrophiles et qui sont différents des émulsionnants ou co-émulsionnants du système émulsionnant.

### Phase lipophile

La phase lipophile appelée aussi phase huileuse ou grasse, est constituée des constituants lipophiles, c'est-à-dire huiles et autres corps lipophiles présents dans la composition, ainsi que de tous additifs lipophiles éventuellement présents. La phase lipophile contient au moins une huile, notamment une huile cosmétique.

La phase lipophile est présente en une quantité d'au moins 50 % en poids par rapport au poids total de la composition. La quantité de phase lipophile peut aller par exemple de 50 à 90 % en poids, de préférence de 60 à 80 % en poids par rapport au poids total de la composition. La phase lipophile peut ne comprendre que des huiles (corps gras liquides) ou elle peut comprebdre un mélange d'huiles et d'autres corps gras. Toutefois, de préférence, la quantité d'huiles est d'au moins 40 % en poids par rapport au poids total de la composition, et de préférence d'au moins 50 % par rapport au poids total de la composition.

De manière caractéristique, la quantité d'huiles hydrocarbonées synthétiques ou minérales, ayant un poids moléculaire supérieur ou égal à 360g/mole est d'au moins 25 % du poids total de la phase lipophile et de préférence d'au moins 30 % du poids total de la phase lipophile. Cette quantité va de préférence de 25 à 100 % et mieux de 30 à 100 % du poids total de la phase lipophile.

On entend par "huile ", un corps gras liquide à la température ambiante (25°C).

On entend par "huile hydrocarbonée", une huile comportant majoritairement des atomes de carbone et d'hydrogène, et éventuellement des groupements ester, éther, fluoré.

Comme huiles hydrocarbonées minérales ou synthétiques de poids moléculaire supérieur ou égal à 360g/mole, utilisables dans la composition de l'invention, on peut citer notamment les esters d'acide gras, de préférence ceux obtenus à partir d'un alcool à chaîne linéaire, ou ramifiée, saturée ou insaturée ayant de 1 à 30 atomes de carbone et d'un acide gras à chaîne linéaire ou ramifiée, ayant de 3 à 30 atomes de carbone, la somme totale d'atomes de carbone des dits esters étant être supérieure ou égale à 24 ; les alcanes; et leurs mélanges.

Comme esters d'acide gras, on peut citer par exemple le palmitate d'ethyl-2 hexyle (ou palmitate d'octyle), l'ethyl-2 hexanoate de cetyle, le myristate d'octyl-2 dodécyle, le stéarate d'isocétyle, l'isostéarate d'isotéaryle, le stéarate d'éthyl-2 hexyle (ou stéarate d'octyle), le neopentanoate d'octyldodecyle, l'isononanoate de cétéaryle, l'isononanoate d'isodécyle, le pentaérythritol de tétraisostéarate, l'isononanoate d'isotridécyle, benzoates d'alcools gras en C12-C15 (Finsolv TN de FINETEX), et leurs mélanges.

Comme alcanes, on peut citer par exemple la vaseline, l'huile de vaseline, l'isoparaffine hydrogéné tel que l'huile de Parléam® commercialisée par la société NOF Corporation (nom INCI ; Hydrogenated Polyisobutene), et leurs mélanges.

Selon un mode plus préféré de réalisation de l'invention, la composition selon l'invention contient un ou plusieurs esters d'acide gras choisis parmi le palmitate d'ethyl-2 hexyle (ou palmitate d'octyle) et l'ethyl-2 hexanoate de cetyle (ou octanoate de cetyle).

La composition peut contenir en outre jusqu'à 75% en poids par rapport au poids des constituants huileux de la phase huileuse, d'une ou plusieurs autres huiles choisies parmi les esters d'acides gras, ayant un poids moléculaire inférieur à 360 g/mole, les éthers, les huiles végétales, les huiles de silicone, les huiles fluorées, volatiles ou non. On peut notamment citer comme huiles de ce type :
- les esters de poids moléculaire inférieur à 360 g/mole, tels que le caprate/caprylate d'éthyl-2 hexyle (ou caprate/caprylate d'octyle), le laurate d'éthyle, le laurate de butyle, le laurate d'hexyle, le laurate d'isohexyle, le laurate d'isopropyle, le myristate de méthyle, le myristate d'éthyle, le myristate de butyle, le myristate d'isobutyle, le myristate d'isopropyle, le monococoate d'éthyl-2 hexyle (ou monococoate d'octyle), le palmitate de méthyle, le palmitate d'éthyle, le palmitate d'isopropyle, le palmitate d'isobutyle, le stéarate de butyle, le stéarate d'isopropyle, le stéarate d'isobutyle, , l'isostéarate d'isopropyle, le pelargonate d'ethyl-2 hexyle (ou pelargonate d'octyle), l'hydroxystéarate d'éthyl-2 hexyle (ou hydroxystéarate d'octyle), le decyl oleate, l' adipate de di-isopropyle, l'adipate de di-éthyl-2 hexyle (ou adipate de di-octyle), l'adipate de diisocetyle, le succinate d'ethyl-2 hexyle (ou succinate d'octyle), le sébacate de diisopropyle, le malate d'ethyl-2 hexyle (ou malate d'octyle), le caprate/caprylate de pentaérythritol, l'hexanoate d'éthyl-2 hexyle (ou hexanoate d'octyle), l'octanoate d'octyldodécyle, le néopentanoate d'isodécyle, le néopentanoate d'isostéaryle, l'isononanoate d'isononyle, l'isononanoate d'isotridécyle, le lactate de lauryle, le lactate de myristyle, le lactate de cétyle, le propionate de myristyle, l'éthyl-2 hexanoate d'éthyl-2 hexyle (ou éthyl-2 hexanoate d'octyle), l'octanoate d'éthyl-2 hexyle (ou octanoate d'octyle, et leurs mélanges, ; lauroyl sarcosinate d'isopropyle (Eldew SL 205 de Unipex), le dicaprylyl carbonate (Cetiol CC de Cognis).
- les éthers tels que le Dicaprylyl éther (Cetiol OE de Cognis).
- les huiles hydrocarbonées d'origine végétale telles que l'huile d'amande douce, l'huile d'avocat, l'huile de ricin, l'huile de coriandre, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore, l'huile de seigle, les triglycérides des acides caprylique/caprique comme ceux vendus par la société Stearineries Dubois ou ceux vendus sous les dénominations Miglyol 810, 812 et 818 par la société Dynamit Nobel ;
- les huiles de silicone, volatiles ou non, comme les polydiméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, les polyméthyl-phénylsiloxanes ;
- les huiles fluorées telles que celles partiellement hydrocarbonées et/ou siliconées comme celles décrites dans le document JP-A-2-295912.
- les huiles hydrocarbonées linéaires ou ramifiés, d'origine minérale, synthétique ou animale, sont choisies parmi l'isohexadecane, l'isododecane, les isoparaffines en C8-9 et C11-C13 (nom CTFA : C8-9 Isoparaffin et C11-13 Isoparaffin).
et leurs mélanges.

Les autres constituants lipophiles pouvant être présents dans la phase lipophile sont par exemple les cires ; les gommes telles que les gommes de silicone (diméthiconol) ; les résines de silicone telles que la trifluorométhyl-C1-4-alkyldimethicone et la Trifluoropropyldimethicone ; les pâtes telles que Petrolatum ; et leurs mélanges.

### Phase aqueuse

La composition selon l'invention comprend une quantité de phase aqueuse inférieure ou égale à 45 % et de préférence inférieure ou égale à 30 % du poids total de la composition, cette quantité pouvant aller par exemple de 10 à 45 % en poids et de préférence de 10 à 30 % en poids par rapport au poids total de la composition. La quantité d'eau dans la phase aqueuse peut aller par exemple de 50 à 100 % en poids par rapport au poids de la phase aqueuse.

Selon un mode préféré de réalisation de l'invention, la composition de l'invention contient au moins un polyol (ou alcools polyhydriques) qui est généralement présent dans la phase aqueuse. Comme polyols, on peut citer par exemple la glycérine ; les glycols comme le propylène glycol, le butylène glycol, l'isoprène glycol et les polyéthylène glycols tels que le PEG-8 ; le sorbitol ; les sucres tels que le glucose, le fructose, le maltose, le lactose, le sucrose ; et leurs mélanges. La quantité de polyols va généralement de 0,1 à 60 % en poids et mieux de 0,5 à 50 % en poids par rapport au poids total de la phase aqueuse.

De manière classique, la phase aqueuse peut contenir, outre l'eau et le ou les polyols, un ou plusieurs solvants hydrosolubles choisis parmi les alcool(s) inférieur(s) hydrosoluble(s). On entend par alcool inférieur, un alcool comportant de 1 à 8 atomes de carbone. Comme alcools inférieurs, on peut citer par exemple l'éthanol, l'isopropanol, le butanol et leurs mélanges. Lorsqu'ils sont présents dans la composition de l'invention, le ou les alcool(s) inférieur(s) hydrosoluble(s) peuvent être en une quantité allant de 0,01 à 40 % en poids et de préférence de 0,01 à 20 % en poids par rapport au poids total de la phase aqueuse.

### Additifs

La composition selon l'invention peut aussi contenir tout adjuvant ou additif habituellement utilisé dans les domaines considérés et notamment dans les domaines cosmétique ou dermatologique. Bien entendu, l'homme de l'art veillera à choisir le ou les éventuels additifs de la composition selon l'invention de manière telle que les propriétés avantageuses attachées intrinsèquement à la composition conforme à l'invention ne soient pas ou substantiellement pas, altérées par l'addition envisagée.

Parmi les adjuvants classiques susceptibles d'être contenus dans la phase aqueuse et/ou dans la phase huileuse des émulsions conformes à l'invention (selon le caractère hydrosoluble ou liposoluble de ces adjuvants), on peut citer notamment les tensioactifs moussants anioniques (tels que lauryl ether sulfate de sodium, alkyl phosphate de sodium, trideceth sulfate de sodium), amphotères (tels que alkyl bétaine, disodium cocoamphodiacetate) ou non ioniques de HLB supérieure à 10 (tels que POE/PPG/POE, Alkylpolyglucoside, polyglyceryl-3 hydroxylauryl ether) ; les conservateurs ; les séquestrants (EDTA) ; les antioxydants ; les parfums ; les matières colorantes telles que les colorants solubles, les pigments et les nacres ; les charges matifiantes, tenseurs, blanchissantes ou exfoliantes ; les filtres solaires ; les actifs cosmétiques ou dermatologiques et agents ayant pour effet d'améliorer les propriétés cosmétiques de la peau, hydrophiles ou lipophiles ; les électrolytes ; les polymères hydrophiles ou lipophiles, anioniques, non ioniques, cationiques ou amphotères, épaississants ou dispersants. Les quantités de ces différents adjuvants sont celles classiquement utilisées dans le domaine considéré, et par exemple de 0,01 à 20 % du poids total de la composition.

Comme actifs utilisables dans la composition de l'invention, on peut citer par exemple, les vitamines hydrosolubles ou liposolubles comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; les antiseptiques ; les actifs anti-bactériens comme le 2,4,4'-trichloro-2'-hydroxy diphényl éther (ou triclosan), le 3,4,4'-trichlorocarbanilide (ou triclocarban) ; les antisébohrréïques ; les antimicrobiens tels que le peroxyde de benzoyle, l'acide salicylique, le triclosan, l'acide azélaïque, la niacine (vit. PP) ; les amincissants tels que la caféine ; les azurants optiques, et tout actif approprié pour le but final de la composition, et leurs mélanges.

La quantité d'actifs dépend du but recherché. Le ou les actifs peuvent être par exemple présents en une concentration allant de 0,001 à 20 %, de préférence de 0,01 à 10 % en poids et mieux de 0,05 à 5 % du poids total de la composition.

Les émulsions selon l'invention sont obtenues généralement par un procédé d'inversion de phase. Ce procédé de préparation consiste à :
- 1) Peser dans un récipient tous les constituants de la composition (à l'exception des matières premières thermosensibles s'il y en a)
- 2) Homogénéiser le mélange, par exemple au moyen d'un Rayneri 350 tours/min, et chauffer en augmentant progressivement la température au moyen d'un bain marie jusqu'à une température supérieure ou égale à la température d'inversion de phase T2, c'est-à-dire jusqu'à l'obtention d'une phase transparente ou translucide (zone de microémulsion ou de phase lamellaire) puis d'une phase blanche plus visqueuse qui indique l'obtention de l'émulsion inverse (E/H).
- 3) Arrêter le chauffage et maintenir l'agitation jusqu'à retour à la température ambiante, en passant par la température d'inversion de phase T1, c'est-à-dire la température à laquelle se forme une émulsion H/E fine.
- 4) Lorsque la température est redescendue en dessous de la zone d'inversion de Phase en Température (T1), additionner éventuellement les matières premières thermosensibles.

On obtient une émulsion H/E stable dont les gouttelettes d'huile sont fines.

Dans la zone de formation d'une microémulsion (mélange translucide), les interactions hydrophiles et hydrophobes sont équilibrées car la tendance du tensioactif est de former aussi bien des micelles directes que des micelles inverses. Par chauffage au-delà de cette zone, il y a formation d'une émulsion E/H (mélange opaque blanc), car le tensioactif favorise la formation d'une émulsion eau dans huile. Puis lors du refroidissement en dessous de la zone d'inversion de phase, l'émulsion devient une émulsion H/E.

L'émulsification par inversion de phase est expliquée en détail dans l'ouvrage T.Förster, W von Rybinski, A.Wadle, Influence of microemulsion phases on the preparation of fine disperse emulsions, Advances in Colloid and interface sciences, 58, 119-149, 1995 cité ici pour référence.

Les compositions selon l'invention se présentent sous forme de crèmes plus ou moins souples, et elles peuvent constituer notamment des compositions cosmétiques ou dermatologiques, par exemple des compositions cosmétiques pour le traitement des matières kératiniques telles que la peau, les muqueuses, les cils, les cheveux et les ongles. Elles peuvent constituer aussi par exemple des compositions de démaquillage et/ou de nettoyage et/ou de soin de la peau, des muqueuses telles que les lèvres, et/ou des cils, des compositions pour le massage de la peau du corps ou du visage, des compositions gommantes (ou exfoliantes) aussi bien pour le visage que pour les mains (lorsque la composition contient des particules exfoliantes), des compositions solaires (protection U.V.), et après-solaires. Elles peuvent constituer encore des compositions de maquillage des matières kératiniques et notamment la peau, les lèvres et les cils, et plus particulièrement comme de fonds de teint, rouges à lèvres, brillants à lèvres (gloss) après addition de pigments et/ou charges appropriés.

Les compositions selon l'invention peuvent être aussi utilisées comme baumes de soin de douche (à rincer, par exemple en massant le produit jusqu'à libération de l'huile, puis en rinçant la peau qui est alors douce et hydratée) ; comme après-shampooings et baumes de soin capillaire ; comme produits de rasage ; comme masques y compris comme masque réparateur après-soleil ; comme cataplasme amincissant sur zone "capitons" (à masser puis à rincer) ; comme baume de massage ; comme baume réparateur lèvres à rincer ; comme baume pour pieds secs. Lors de ces utilisations, le produit est ensuite rincé.

Lorsque la composition est un produit exfoliant (appelé aussi produit descincrustant), l'utilisation consiste à appliquer le produit sur le visage ou les mains ou le corps, à frotter une minute ou deux puis à rincer. La peau est alors lisse, douce et désincrustée.

L'invention a encore pour objet l'utilisation cosmétique de la composition telle que définie ci-dessus comme produit de soin de la peau, comme produit d'hygiène, comme produit capillaire, comme produit solaire et comme produit de maquillage.

Un autre objet de l'invention est un procédé de traitement cosmétique d'une matière kératinique, telle que la peau, du cuir chevelu, des cheveux, des cils, des sourcils, des ongles ou des muqueuses, caractérisé par le fait qu'on applique sur la matière kératinique, une composition telle que définie ci-dessus.

La matière kératinique est de préférence la peau.

Les compositions selon l'invention peuvent être utilisées différemment en fonction des applications souhaitées, par exemple :
- appliquée seulement (sans rinçage) comme produit de soin du visage ou du corps, ou de maquillage de la peau, des lèvres ou des yeux, ou comme produit solaire par exemple,
- essuyée, rincée à l'eau ou avec un tonique comme produit démaquillant
- rincée à l'eau comme produit de soin capillaire après shampooing.

Les exemples indiqués ci-dessous permettront de mieux comprendre l'invention sans toutefois présenter un caractère limitatif. Les quantités indiquées sont en % en poids sauf mention contraire. Les noms sont en noms chimiques et en noms CTFA selon les composés. Dans certains exemples, les températures T1 et T2 correspondant aux températures bordant la zone d'inversion de phase sont indiquées.

Les exemples réalisés ont été soumis à des mesures de viscosité permettant de mettre en évidence leur stabilité dans le temps.

Les matières premières utilisées dans les exemples sont les suivantes :
(1) EUMULGIN BA 10® commercialisé par Cognis = Alcool béhénylique oxyéthyléné (10 OE)
(2) ISOPROPYL PALMITATE® commercialisé par Cognis = palmitate d'isopropyle
(3) STEARATE D'ISOCETYLE® commercialisé par Stéarineries Dubois = : isocetyl stearate
(4) CETIOL OE® commercialisé par Cognis = : dicaprylyl éther
(5) CEGESOFT C 24® commercialisé par Cognis ou CERAPHYL 368® commercialisé par ISP = : Palmitate d'éthyl 2 hexyle.

### Exemples 1 à 5 selon l'invention

| Composition | Exemple 1 | Exemple 2 |
|---|---|---|
| EUMULGIN BA 10® (1) | 5 | 5 |
| STEARATE D'ISOCETYLE® (3) *(PM 509 g*/*mole)* | 11,55 | 16,17 |
| CETIOL OE® (4) *(PM 240 g*/*mole)* | 23,45 | 46,9 |
| CEGESOFT C 24® (5) *(PM* 368 *g*/*mole)* | 35 | 0 |
| Propyl paraben | 0,15 | 0,15 |
| Methyl paraben | 0,15 | 0,15 |
| Chlorhexidine digluconate | 0,25 | 0,25 |
| Glycérine | 10 | 10 |
| Eau permutée | 14,45 | 14,45 |
| total | 100 | 100 |
| % Total en tensioactifs (TA) | 5 | 5 |
| % Total de constituants huileux | 70 | 70 |
| Rapport TA/constituants huileux | 0,07 | 0,07 |
| % huile de PM ≥ 360g/mol par rapport à la totalité de constituants huileux | 66,5 | 76,9 |
| T1 (°C) | 75 | 72 |
| T2 (°C) | 81 | 78 |
| Aspect | Emulsion opaque blanche, très lisse, brillante | Emulsion opaque blanche très lisse, brillante |
| pH | 5,7 | 6 |
| Aspect macroscopique) après 2 mois 45°C | Homogène | Homogène |
| Viscosité (Pa.s) à 24 h à température ambiante. à t10 minutes | 6,9 | 5,4 |
| Viscosité (Pa.s) après 2 mois 45°C à t10 minutes | 4,7 | 4 |
| Variation de viscosité par rapport à 24h à température ambiante | - 32 % | - 26% |
| Conclusion | Variation de viscosité acceptable | Variation de viscosité acceptable |

| Composition | Exemple 3 | Exemple 4 |
|---|---|---|
| EUMULGIN BA 10® (1) | 5 | 5 |
| STEARATE D'ISOCETYLE® (3) *(PM 509 g*/*mole)* | 0 | 70 |
| CEGESOFT C 24® (5) *(PM 368 g*/*mole)* | 70 | 0 |
| Propyl paraben | 0,15 | 0,15 |
| Methyl paraben | 0,15 | 0,15 |
| Chlorhexidine digluconate | 0,25 | 0,25 |
| Glycérine | 10 | 10 |
| Eau permutée | 14,45 | 14,45 |
| total | 100 | 100 |
| % Total en tensioactifs (TA) | 5 | 5 |
| % Total de constituants huileux | 70 | 70 |
| Rapport TA/constituants huileux | 0,07 | 0,07 |
| % huile de PM ≥ 360g/mol par rapport à la totalité de constituants huileux | 100 | 100 |
| T1 (°C) | 73 | 77 |
| T2 (°C) | 85 | 85 |
| Aspect | Emulsion opaque/ translucide homogène, lisse | Emulsion blanche |
| pH | 5,7 | 6 |
| Aspect macroscopique) après 2 mois 45°C | Homogène | Homogène |
| Viscosité (Pa.s) à 24 h à température ambiante. à t10 minutes | 6,9 | 7,3 |
| Viscosité (Pa.s) après 2 mois 45°C à t10 minutes | 6,6 | 7,8 |
| Variation de viscosité par rapport à 24h à température ambiante | - 4 % | + 6% |
| Conclusion | Très faible variation de viscosité. | Très faible variation de viscosité |

| Composition | Exemple 5 |
|---|---|
| EUMULGIN BA 10® (1) | 5 |
| CEGESOFT C 24® (5) *(PM 368 g*/*mole)* | 64 |
| Propyl paraben | 0,15 |
| Methyl paraben | 0,15 |
| Chlorhexidine digluconate | 0,25 |
| Glycérine | 10 |
| Eau permutée | 18,45 |
| Ethanol | 2 |
| total | 100 |
| % Total en tensioactifs (TA) | 5 |
| % Total de constituants huileux | 64 |
| Rapport TA/constituants huileux | 0,078 |
| % huile de PM ≥ 360g/mol par rapport à la totalité de constituants huileux | 100 |
| T1 (°C) | 73 |
| T2 (°C) | 85 |
| Aspect | Emulsion opaque blanche, très lisse, brillante |
| pH | 6,7 |
| Aspect macroscopique) après 2 mois 45°C | Homogène |
| Viscosité (Pa.s) à 24 h à température ambiante. à t10 minutes | 5,9 |
| Viscosité (Pa.s) après 2 mois 45°C à t10 minutes | 7,1 |
| Variation de viscosité par rapport à 24h à température ambiante | 20 % |
| Conclusion | Variation de viscosité acceptable |

### III. Exemples comparatifs :

| Composition | Exemple comparatif 1 | Exemple comparatif 2 |
|---|---|---|
| EUMULGIN BA 10® (1) | 5 | 5 |
| ISOPROPYL PALMITATE® (2) *(PM 298 g*/*mole)* | 0 | 70 |
| CETIOL OE® (4) *(PM 240 g*/*mole)* | 70 | 0 |
| Propyl paraben | 0,15 | 0,15 |
| Methyl paraben | 0,15 | 0,15 |
| Chlorhexidine digluconate | 0,25 | 0,25 |
| Glycérine | 10 | 10 |
| Eau permutée | 14,45 | 14,45 |
| total | 100 | 100 |
| % Total en tensioactifs (TA) | 5 | 5 |
| % Total de constituants huileux | 70 | 70 |
| Rapport TA/constituants huileux | 0,07 | 0,07 |
| % huile de PM ≥ 360g/mol par rapport à la totalité de constituants huileux | 0 | 0 |
| T1 (°C) | Néant | néant |
| T2 (°C) | Supérieure à 95°C | Supérieure à 95°C |
| Aspect | L'émulsion ne se fait pas : deux phases | L'émulsion ne se fait pas : deux phases |
| Conclusion | Impossible de faire l'émulsion : le PM de l'huile (240 g/mol) est trop faible | Impossible de faire l'émulsion : le PM de l'huile (298 g/mol) est trop faible |

Ces exemples comparatifs montrent qu'en absence d'huile de PM > 360 g/mole, on ne peut pas obtenir d'émulsions.

| Composition | Exemple comparatif 3 | Exemple comparatif 4 |
|---|---|---|
| EUMULGIN BA 10® (1) | 5 | 5 |
| ISOPROPYL PALMITATE® (2) *(PM 298 g*/*mole)* | 35 | 20 |
| STEARATE D'ISOCETYLE® (3) *(PM 509 g*/*mole)* | 11,55 | 16,5 |
| CETIOL OE® (4) *(PM 240 g*/*mole)* | 23,45 | 33,50 |
| Propyl paraben | 0,15 | 0,15 |
| Methyl paraben | 0,15 | 0,15 |
| Chlorhexidine digluconate | 0,25 | 0,25 |
| Glycérine | 10 | 10 |
| Eau permutée | 14,45 | 14,45 |
| total | 100 | 100 |
| % Total en tensioactifs (TA) | 5 | 5 |
| % Total de constituants huileux | 70 | 70 |
| Rapport TA/constituants huileux | 0,07 | 0,07 |
| % huile de PM ≥ 360g/mol par rapport à la totalité de constituants huileux | 16,5% | 23,6% |
| T1 (°C) | 68 | 70 |
| T2 (°C) | 75 | 75 |
| Aspect | Emulsion translucide/ opaque, lisse | Emulsion translucide/opaque très lisse |
| pH | 6 | 5,7 |
| Aspect macroscopique) après 2 mois 45°C | Légèrement granuleuse. | Légèrement granuleuse. |
| Viscosité (Pa.s) à 24 h à température ambiante. à t10 minutes | 5,7 | 5,9 |
| Viscosité (Pa.s) après 2 mois 45°C à t10 minutes | 3,1 | 3,8 |
| Variation de viscosité par rapport à 24h à température ambiante | - 46% | - 35% |
| Conclusion | Variation de viscosité trop importante | Variation de viscosité trop importante |

Ces exemples comparatifs montrent qu'en absence d'une quantité suffisante d'huile de PM > 360 g/mole, les émulsions obtenues présentent une viscosité qui varient trop au cours du temps pour que les émulsions puissent être considérées comme des émulsions stables.

## Revendications

1. Composition pour application topique sous forme d'émulsion huile-dans-eau, **caractérisée par le fait qu'**elle comporte :
- Une phase lipophile (A) présente en une quantité d'au moins 50 % en poids par rapport au poids total de la composition, la phase lipophile comprenant au moins 25 % en poids d'une ou plusieurs huiles hydrocarbonées synthétiques ou minérales, ayant un poids moléculaire supérieur ou égal à 360 g/mole, par rapport au poids total de la phase lipophile,
- une phase aqueuse (C) présente en une quantité inférieure ou égale à 45 % en poids par rapport au poids total de la composition,
- un système émulsionnant (B) présent en une quantité de 2 à 20 % en poids par rapport au poids total de la composition et comprenant au moins un émulsionnant ayant un HLB allant de 8 à 18, choisi parmi les alcools gras éthoxylés, les acides gras éthoxylés, les glycérides partiels d'acides gras ethoxylés, les triglycérides d'acides gras polyglycérolés et leurs dérivés éthoxylés, et leurs mélanges,
- le rapport système émulsionnant (B) / phase lipophile (A) allant de 0,04 à 0,2.

2. Composition selon la revendication 1, **caractérisée par le fait qu'**elle est susceptible d'être obtenue selon la technique d'émulsification par inversion de phase.

3. Composition selon la revendication 1 ou 2, **caractérisée par le fait que** la taille moyenne des gouttelettes de phase huileuse D[4,3] va de 0,09 µm à 4 µm.

4. Composition selon l'une quelconque des revendications précédentes, **caractérisée par le fait qu'**elle présente une viscosité à 25°C, égale ou supérieure à 1 Pa.s.

5. Composition selon l'une quelconque des revendications précédentes, **caractérisée par le fait que** l'émulsionnant est choisi parmi les produits d'addition d'oxyde d'éthylène avec l'alcool laurylique ; les produits d'addition d'oxyde d'éthylène avec l'alcool béhénylique ; les produits d'addition d'oxyde d'éthylène avec l'alcool cétéarylique ; les produits d'addition d'oxyde d'éthylène avec l'alcool cétylique ; les produits d'addition d'oxyde d'éthylène avec l'alcool stéarylique ; les produits d'addition d'oxyde d'éthylène avec l'alcool isostéarylique , les produits d'addition d'oxyde d'éthylène avec les acides laurique, palmitique, stéarique ou béhénique, et leurs mélanges.

6. Composition selon la revendication précédente, **caractérisée par le fait que** l'émulsionnant est le beheneth-10.

7. Composition selon l'une quelconque des revendications précédentes, **caractérisée par le fait que** le système émulsionnant contient en outre un ou plusieurs co-émulsionnants choisis parmi les alcools gras ayant 8 à 30 atomes de carbone ; les acides gras ayant 8 à 30 atomes de carbone ; les esters gras de glycérol ; leurs dérivés oxyéthylénés comportant 2 à 8 groupes oxyde d'éthylène, et leurs mélanges.

8. Composition selon l'une quelconque des revendications précédentes, **caractérisée par le fait que** la quantité de système émulsionnant va de 3 à 16 % en poids par rapport au poids total de la composition.

9. Composition selon l'une quelconque des revendications précédentes, **caractérisée par le fait que** la quantité de phase lipophile va de 50 à 90 % en poids par rapport au poids total de la composition.

10. Composition selon l'une quelconque des revendications précédentes, **caractérisée par le fait que** les huiles hydrocarbonées synthétiques ou minérales de poids moléculaire supérieur ou égal à 360g/mole sont choisies parmi les esters d'acide gras obtenus à partir d'un alcool à chaîne linéaire, ou ramifiée, saturée ou insaturée ayant de 1 à 24 atomes de carbone et d'un acide gras à chaîne linéaire ou ramifiée, ayant de 3 à 24 atomes de carbone, la somme totale d'atomes de carbone des dits esters étant être supérieure ou égale à 24 ; les alcanes ; et leurs mélanges.

11. Composition selon l'une quelconque des revendications précédentes, **caractérisée par le fait que** les huiles hydrocarbonées synthétiques ou minérales de poids moléculaire supérieur ou égal à 360g/mole sont choisies parmi le palmitate d'ethyl-2 hexyle, l'ethyl-2 hexanoate de cetyle, le myristate d'octyl-2 dodécyle, le stéarate d'isocétyle, l'isostéarate d'isotéaryle, le stéarate d'éthyl-2 hexyle, le neopentanoate d'octyldodecyle, l'isononanoate de cétéaryle, l'isononanoate d'isodécyle, le pentaérythritol de tétraisostéarate, l'isononanoate d'isotridécyle, benzoates d'alcools gras en C12-C15, la vaseline, l'huile de vaseline, l'isoparaffine hydrogéné, et leurs mélanges.

12. Composition selon l'une quelconque des revendications précédente, **caractérisée par le fait que** la quantité d'huiles hydrocarbonées synthétiques ou minérales ayant un poids moléculaire supérieur ou égal à 360g/mole va de 25 à 100 % du poids des constituants huileux de la phase huileuse.

13. Composition selon l'une quelconque des revendications précédentes, **caractérisée par le fait qu'**elle contient au moins un polyol.

14. Composition selon la revendication précédente, **caractérisée par le fait que** le polyol est choisi parmi la glycérine ; les glycols comme le propylène glycol, le butylène glycol, l'isoprène glycol et les polyéthylène glycols tels que le PEG-8 ; le sorbitol ; les sucres tels que le glucose, le fructose, le maltose, le lactose, le sucrose ; et leurs mélanges.

15. Composition selon la revendication 13 ou 14, **caractérisée par le fait que** la quantité de polyol(s) va de 5 à 20 % en poids par rapport au poids total de la composition.

16. Composition selon l'une quelconque des revendications précédentes, **caractérisée par le fait qu'**elle constitue une composition cosmétique ou dermatologique.

17. Composition selon l'une quelconque des revendications 1 à 15, **caractérisée par le fait qu'**elle constitue une composition de démaquillage et/ou de nettoyage de la peau, des lèvres et/ou des cils, une composition pour le massage de la peau du corps et/ou du visage, une composition exfoliante, une composition solaire et après-solaire, un baume de soin de douche, une composition après- shampooing, un baume de soin capillaire, un produit de rasage, un masque, un cataplasme amincissant, un baume de massage, un baume réparateur lèvres, un baume pour pieds secs.

18. Utilisation cosmétique de la composition selon l'une quelconque des revendications 1 à 15, pour le traitement cosmétique des matières kératiniques.

19. Procédé de traitement cosmétique d'une matière kératinique, **caractérisé par le fait qu'**on applique sur la matière kératinique, une composition selon l'une quelconque des revendications 1 à 15.

20. Procédé de préparation d'une composition selon l'une quelconque des revendications 1 à 15, consistant à :
- 1) Peser dans un récipient tous les constituants de la composition (à l'exception des matières premières thermosensibles)
- 2) Homogénéiser le mélange, et chauffer en augmentant progressivement la température au moyen d'un bain marie jusqu'à une température supérieure ou égale à la température d'inversion de phase T2,
- 3) Arrêter le chauffage et maintenir l'agitation jusqu'à retour à la température ambiante, en passant par la température d'inversion de phase T1,
- 4) Lorsque la température est redescendue en dessous de la zone d'inversion de Phase en Température (T1), additionner éventuellement les matières premières thermosensibles.

## Claims

1. Composition for topical application in the form of an oil-in-water emulsion, **characterized in that** it contains:
- a lipophilic phase (A) present in an amount of at least 50% by weight relative to the total weight of the composition, the lipophilic phase comprising at least 25% by weight of one or more synthetic or mineral hydrocarbon-based oils having a molecular weight greater than or equal to 360 g/mol, relative to the total weight of the lipophilic phase,
- an aqueous phase (C) present in an amount of less than or equal to 45% by weight relative to the total weight of the composition,
- an emulsifying system (B) present in an amount of 2 to 20% by weight relative to the total weight of the composition and comprising at least one emulsifier having an HLB ranging from 8 to 18, chosen from ethoxylated fatty alcohols, ethoxylated fatty acids, partial glycerides of ethoxylated fatty acids, polyglycerolated fatty acid triglycerides and ethoxylated derivatives thereof and mixtures thereof,
- the emulsifying system (B)/lipophilic phase (A) ratio ranging from 0.04 to 0.2.

2. Composition according to Claim 1, **characterized in that** it can be obtained according to the phase inversion emulsification technique.

3. Composition according to Claim 1 or 2, **characterized in that** the mean size of the droplets of oily phase D[4.3] ranges from 0.09 µm to 4 µm.

4. Composition according to any one of the preceding claims, **characterized in that** it has a viscosity at 25°C of greater than or equal to 1 Pa.s.

5. Composition according to any one of the preceding claims, **characterized in that** the emulsifier is chosen from the addition products of ethylene oxide with lauryl alcohol; the addition products of ethylene oxide with behenyl alcohol; the addition products of ethylene oxide with cetearyl alcohol; the addition products of ethylene oxide with cetyl alcohol; the addition products of ethylene oxide with stearyl alcohol; the addition products of ethylene oxide with isostearyl alcohol; the addition products of ethylene oxide with lauric acid, palmitic acid, stearic acid or behenic acid, and mixtures thereof.

6. Composition according to the preceding claim, **characterized in that** the emulsifier is beheneth-10.

7. Composition according to any one of the preceding claims, **characterized in that** the emulsifying system also contains one or more coemulsifier(s) chosen from fatty alcohols having 8 to 30 carbon atoms; fatty acids having 8 to 30 carbon atoms; fatty esters of glycerol; oxyethylenated derivatives thereof containing 2 to 8 ethylene oxide groups, and mixtures thereof.

8. Composition according to any one of the preceding claims, **characterized in that** the amount of emulsifying system ranges from 3 to 16% by weight relative to the total weight of the composition.

9. Composition according to any one of the preceding claims, **characterized in that** the amount of lipophilic phase ranges from 50 to 90% by weight relative to the total weight of the composition.

10. Composition according to any one of the preceding claims, **characterized in that** the synthetic or mineral hydrocarbon-based oils having a molecular weight of greater than or equal to 360 g/mol are chosen from fatty acid esters obtained from an alcohol comprising a saturated or unsaturated, linear or branched chain having from 1 to 24 carbon atoms and from a fatty acid comprising a linear or branched chain having from 3 to 24 carbon atoms, the total sum of carbon atoms of said esters being greater than or equal to 24; alkanes, and mixtures thereof.

11. Composition according to any one of the preceding claims, **characterized in that** the synthetic or mineral hydrocarbon-based oils having a molecular weight of greater than or equal to 360 g/mol are chosen from 2-ethylhexyl palmitate, cetyl 2-ethylhexanoate, 2-octyldodecyl myristate, isocetyl stearate, isostearyl isostearate, 2-ethylhexyl stearate, octyldodecyl neopentanoate, cetearyl isononanoate, isodecyl isononanoate, pentaerythritol tetraisostearate, isotridecyl isononanoate, C₁₂-C₁₅ fatty alcohol benzoates, petroleum jelly, liquid petroleum jelly, hydrogenated isoparaffin, and mixtures thereof.

12. Composition according to any one of the preceding claims, **characterized in that** the amount of synthetic or mineral hydrocarbon-based oils having a molecular weight of greater than or equal to 360 g/mol ranges from 25 to 100% of the weight of the oily constituents of the oily phase.

13. Composition according to any one of the preceding claims, **characterized in that** it contains at least one polyol.

14. Composition according to the preceding claim, **characterized in that** the polyol is chosen from glycerol; glycols such as propylene glycol, butylene glycol, isoprene glycol and polyethylene glycols such as PEG-8; sorbitol; sugars such as glucose, fructose, maltose, lactose or sucrase; and mixtures thereof.

15. Composition according to Claim 13 or 14, **characterized in that** the amount of polyol(s) ranges from 5 to 20% by weight relative to the total weight of the composition.

16. Composition according to any one of the preceding claims, **characterized in that** it constitutes a cosmetic or dermatological composition.

17. Composition according to any one of Claims 1 to 15, **characterized in that** it constitutes a makeup-removing composition and/or a cleansing composition for the skin, the lips and/or the eyelashes, a composition for massaging facial skin and/or body skin, an exfoliating composition, an antisun composition or an aftersun composition, a shower care balm, a conditioner composition, a hair care balm, a shaving product, a mask, a slimming poultice, a massage balm, a lip repairing balm, a balm for dry feet.

18. Cosmetic use of the composition according to any one of Claims 1 to 15, for the cosmetic treatment of keratin materials.

19. Process for the cosmetic treatment of a keratin material, **characterized in that** a composition according to any one of Claims 1 to 15 is applied to the keratin material.

20. Process for preparing a composition according to any one of Claims 1 to 15, consisting in:
- 1) Weighing out, into a container, all the constituents of the composition (with the exception of the thermosensitive starting materials).
- 2) Homogenizing the mixture, and heating by gradually increasing the temperature, by means of a water bath, to a temperature greater than or equal to the phase inversion temperature T2.
- 3) Stopping the heating and maintaining the stirring until ambient temperature is again reached, passing through the phase inversion temperature T1.
- 4) When the temperature has again dropped below the phase inversion temperature (T1) region, optionally adding the thermosensitive starting materials.

## Patentansprüche

1. Zusammensetzung für die topische Anwendung in Form einer Öl-in-Wasser-Emulsion, **dadurch gekennzeichnet, dass** sie enthält:
- eine lipophile Phase (A), die in einer Menge von mindestens 50 Gew.-%, bezogen auf das Gesamtgewicht der Zusammensetzung, enthalten ist, wobei die lipophile Phase mindestens 25 Gew.-% eines oder mehrerer, synthetischer oder mineralischer Kohlenwasserstofföle mit einer Molmasse von 360 g/mol oder darüber, bezogen auf das Gesamtgewicht der lipophilen Phase, enthält,
- eine wässrige Phase (C), die in einer Menge von 45 Gew.-% oder darunter, bezogen auf das Gesamtgewicht der Zusammensetzung, enthalten ist,
- ein Emulgatorsystem (B), das in einer Menge von 2 bis 20 Gew.-%, bezogen auf das Gesamtgewicht der Zusammensetzung, enthalten ist und das mindestens einen Emulgator mit einem HLB-Wert im Bereich von 8 bis 18 umfasst, der unter den ethoxylierten Fettalkoholen, ethoxylierten Fettsäuren, partiellen Glyceriden von Fettsäuren, die ethoxyliert sind, Triglyceriden von Fettsäuren, die mehrfach mit Glycerin verethert sind, und deren ethoxylierten Derivaten und deren Gemischen ausgewählt ist,
- wobei das Verhältnis Emulgatorsystem (B) / lipophile Phase (A) im Bereich von 0,04 bis 0,2 liegt.

2. Zusammensetzung nach Anspruch 1, **dadurch gekennzeichnet, dass** sie nach dem Verfahren des Emulgierens unter Phaseninversion erhältlich ist.

3. Zusammensetzung nach Anspruch 1 oder 2, **dadurch gekennzeichnet, dass** die mittlere Größe der Tröpfchen der Ölphase D[4,3] im Bereich von 0,09 bis 4 µm liegt.

4. Zusammensetzung nach einem der vorhergehenden Ansprüche, **dadurch gekennzeichnet, dass** sie bei 25 °C eine Viskosität von 1 Pa·s oder darüber aufweist.

5. Zusammensetzung nach einem der vorhergehenden Ansprüche, **dadurch gekennzeichnet, dass** der Emulgator unter den Additionsprodukten von Ethylenoxid und Laurylalkohol; den Additionsprodukten von Ethylenoxid und Behenylalkohol; den Additionsprodukten von Ethylenoxid und Cetearylalkohol; den Additionsprodukten von Ethylenoxid und Cetylalkohol; den Additionsprodukten von Ethylenoxid und Stearylalkohol; den Additionsprodukten von Ethylenoxid und Isostearylalkohol; den Additionsprodukten von Ethylenoxid und Laurinsäure, Palmitinsäure, Stearinsäure oder Behensäure; und deren Gemischen ausgewählt ist.

6. Zusammensetzung nach dem vorhergehenden Anspruch, **dadurch gekennzeichnet, dass** es sich bei dem Emulgator um das Beheneth-10 handelt.

7. Zusammensetzung nach einem der vorhergehenden Ansprüche, **dadurch gekennzeichnet, dass** das Emulgatorsystem ferner einen oder mehrere Coemulgatoren enthält, die unter den Fettalkoholen mit 8 bis 30 Kohlenstoffatomen; den Fettsäuren mit 8 bis 30 Kohlenstoffatomen; den Glycerylfettsäureestern; deren ethoxylierten Derivaten mit 2 bis 8 Ethylenoxidgruppen und deren Gemischen ausgewählt sind.

8. Zusammensetzung nach einem der vorhergehenden Ansprüche, **dadurch gekennzeichnet, dass** der Mengenanteil des Emulgatorsystems im Bereich von 3 bis 16 Gew.-%, bezogen auf das Gesamtgewicht der Zusammensetzung, liegt.

9. Zusammensetzung nach einem der vorhergehenden Ansprüche, **dadurch gekennzeichnet, dass** der Mengenanteil der lipophilen Phase im Bereich von 50 bis 90 Gew.-%, bezogen auf das Gesamtgewicht der Zusammensetzung, liegt.

10. Zusammensetzung nach einem der vorhergehenden Ansprüche, **dadurch gekennzeichnet, dass** die synthetischen oder mineralischen Kohlenwasserstofföle mit einer Molmasse vom mindestens 360 g/mol unter den Fettsäureestern, die ausgehend von einem gesättigten oder ungesättigten Alkohol mit gerader oder verzweigter Kette, der 1 bis 24 Kohlenstoffatomen aufweist, und einer Fettsäure mit gerader oder verzweigter Kette, die 3 bis 24 Kohlenstoffatomen aufweist, gebildet sind, wobei die Gesamtzahl der Kohlenstoffatome dieser Ester größer oder gleich 24 ist; den Alkanen; und deren Gemischen ausgewählt sind.

11. Zusammensetzung nach einem der vorhergehenden Ansprüche, **dadurch gekennzeichnet, dass** die synthetischen oder mineralischen Kohlenwasserstofföle mit einer Molmasse vom mindestens 360 g/mol unter 2-Ethylhexylpalmitat, Cetyl-2-ethylhexanoat, 2-Octyldodecylmyristat, Isocetylstearat, Isostearylisostearat, 2-Ethylhexylstearat, Octyldodecylneopentanoat, Cetearylisononanoat, Isodecylisononanoat, Pentaerythrityltetraisostearat, Isotridecylisononanoat, Benzoaten von C₁₂₋₁₅-Fettalkoholen, Vaseline, Vaselineöl, hydriertem Isoparaffin und deren Gemischen ausgewählt sind.

12. Zusammensetzung nach einem der vorhergehenden Ansprüche, **dadurch gekennzeichnet, dass** der Mengenanteil der synthetischen oder mineralischen Kohlenwasserstofföle mit einer Molmasse vom mindestens 360 g/mol im Bereich von 25 bis 100 % des Gewichts der Ölbestandteile der Ölphase liegt.

13. Zusammensetzung nach einem der vorhergehenden Ansprüche, **dadurch gekennzeichnet, dass** sie mindestens ein Polyol enthält.

14. Zusammensetzung nach dem vorhergehenden Anspruch, **dadurch gekennzeichnet, dass** das Polyol unter Glycerin; Glycolen wie Propylenglycol, Butylenglycol, Isoprenglycol und Polyethylenglycolen, z.B. PEG-8; Sorbit; Zuckern, wie Glucose, Fructose, Maltose, Lactose, Saccharose; und deren Gemischen ausgewählt ist.

15. Zusammensetzung nach Anspruch 13 oder 14, **dadurch gekennzeichnet, dass** der Mengenanteil an Polyol(en) im Bereich von 5 bis 20 Gew.-%, bezogen auf das Gesamtgewicht der Zusammensetzung, liegt.

16. Zusammensetzung nach einem der vorhergehenden Ansprüche, **dadurch gekennzeichnet, dass** es sich um eine kosmetische oder dermatologische Zusammensetzung handelt.

17. Zusammensetzung nach einem der Ansprüche 1 bis 15, **dadurch gekennzeichnet, dass** es sich um eine Zusammensetzung zum Abschminken und/oder für die Reinigung der Haut, der Lippen und/ oder der Wimpern, ein Produkt für die Massage der Haut des Körpers und/oder des Gesichts, eine exfoliative Zusammensetzung, ein Sonnenschutz- und After-Sun-Produkt, ein pflegendes Duschprodukt, ein Produkt, das nach der Haarwäsche angewandt wird, einen Pflegebalsam für das Haar, ein Produkt für die Rasur, eine Maske, ein schlankermachendes Kataplasma, einen Massagebalsam, einen stärkenden Lippenbalsam, einen Balsam für trockene Füße handelt.

18. Kosmetische Verwendung der Zusammensetzung nach einem der Ansprüche 1 bis 15 für die kosmetische Behandlung von Keratinsubstanzen.

19. Verfahren zur kosmetischen Behandlung einer Keratinsubstanz, **dadurch gekennzeichnet, dass** auf die Keratinsubstanz eine Zusammensetzung nach einem der Ansprüche 1 bis 15 aufgetragen wird.

20. Verfahren zur Herstellung einer Zusammensetzung nach einem der Ansprüche 1 bis 15, das darin besteht:
- 1) alle Bestandteile der Zusammensetzung (mit Ausnahme der wärmeempfindlichen Rohstoffe) werden in einem Gefäß eingewogen,
-2) das Gemisch wird homogenisiert und man erwärmt, indem die Temperatur mit Hilfe eines Wasserbades allmählich bis auf eine Temperatur gleich oder über der Phaseninversionstemperatur T2 erhöht wird,
- 3) man beendet das Erwärmen und rührt bis zum Zurückkommen auf Raumtemperatur weiter, wobei hierbei die Phaseninversionstemperatur T1 durchlaufen wird,
- 4) wenn die Temperatur unter den Bereich der Phaseninversionstemperatur (T1) gefallen ist, arbeitet man gegebenenfalls die wärmeempfindlichen Rohstoffe ein.
